# EUROPÄISCHE PATENTSCHRIFT

(11) **EP 3 607 923 B1**
(45) Veröffentlichungstag und Bekanntmachung des Hinweises auf die Patenterteilung: **22.01.2025**
(21) Anmeldenummer: 18188502.1
(22) Anmeldetag: 10.08.2018
(51) Int. Cl.: A61F 9/06

(54) **VERFAHREN ZU EINEM BETRIEB EINER BLENDSCHUTZVORRICHTUNG**
METHOD FOR OPERATING AN ANTI-GLARE SCREEN
PROCÉDÉ DE FONCTIONNEMENT D'UN DISPOSITIF DE PROTECTION CONTRE L'ÉBLOUISSEMENT

(43) Veröffentlichungstag der Anmeldung: 12.02.2020
(73) Patentinhaber: Optrel Holding AG, 9630 Wattwil (CH)
(72) Erfinder: YANG, Sheng, 9500 Wil (CH); LANDOLT, Marco, 8752 Näfels (CH); HEUSSER, Jonathan, 8713 Uerikon (CH)
(74) Vertreter: Daub, Thomas

(56) Entgegenhaltungen:
- WO-A1-98/57606
- US-A1- 2016 346 131
- US-A1- 2017 143 549
- US-B2- 7 161 135

## Beschreibung

### Stand der Technik

Die Erfindung betrifft ein Verfahren zu einem Betrieb einer Blendschutzvorrichtung.

Es sind bereits Verfahren zu einem Betrieb einer Blendschutzvorrichtung mit einem aktiven optischen Blendschutzfilter und mit zumindest einer Sensoreinheit bekannt, wobei mittels der Sensoreinheit in einem ersten Erfassungsschritt eine Lichtänderung erkannt wird und anschließend abhängig von einer Intensität der Lichtänderung eine davon abhängige Ansteuerung einer Durchlässigkeit des Blendschutzfilters erfolgt, wobei der Blendschutzfilter bei einer Lichtänderung größer gleich einer eingestellten Sensitivität abgedunkelt wird.

Um aktiv schaltbare Blendschutzvorrichtungen auch zum Schleifen verwenden zu können, wird bislang insbesondere ein Bedienelement, wie beispielsweise ein Taster, insbesondere auf einer Außenseite der Blendschutzvorrichtung verwendet. Durch das manuelle betätigen einer Steuereinheit wird der aktive optische Blendschutzfilter ausgeschaltet. Dadurch bleibt der Blendschutzfilter immer im offenen Zustand.

Ferner ist aus der US 2017/0143549 A1 bereits eine Sensoreinheit bekannt, welche einen nicht-optischen Sensor dazu verwenden kann, zu erfassen, ob ein Schweißvorgang, ein Schneidevorgang und/oder ein Schleifvorgang durchgeführt wird.

Des Weiteren ist aus der US 7,161,135 B2 bereits ein Verfahren zu einem Betrieb einer Blendschutzvorrichtung mit einem aktiven optischen Blendschutzfilter und mit zumindest einer Sensoreinheit bekannt, wobei mittels der Sensoreinheit in einem ersten Erfassungsschritt eine Lichtänderung erkannt wird und abhängig von einer Intensität der Lichtänderung eine davon abhängige Ansteuerung einer Durchlässigkeit des Blendschutzfilters erfolgt, wobei der Blendschutzfilter bei einer Lichtänderung größer gleich einer eingestellten Sensitivität abgedunkelt wird, wobei nach einer Abdunklung des Blendschutzfilters in einem zweiten Erfassungsschritt ein Wert einer mittels der Sensoreinheit erfassten Lichtänderung in einem definierten Spektralbereich mit einem Grenzwert verglichen und bei einer Unterschreitung des Grenzwerts der Blendschutzfilter aufgemacht wird.

Die Aufgabe der Erfindung besteht insbesondere darin, ein gattungsgemäßes Verfahren, bei welchem zwischen einem Schweißbetrieb und einem Schleifbetrieb unterschieden werden kann, mit verbesserten Eigenschaften hinsichtlich einer Betriebssicherheit bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung geht aus von eine Verfahren zu einem Betrieb einer Blendschutzvorrichtung mit einem aktiven optischen Blendschutzfilter und mit zumindest einer Sensoreinheit, wobei mittels der Sensoreinheit in einem ersten Erfassungsschritt eine Lichtänderung erkannt wird und, insbesondere anschließend, abhängig von einer Intensität der Lichtänderung eine davon abhängige Ansteuerung einer Durchlässigkeit des Blendschutzfilters erfolgt, wobei der Blendschutzfilter bei einer Lichtänderung größer gleich einer eingestellten Sensitivität abgedunkelt wird, wobei nach einer Abdunklung des Blendschutzfilters in einem zweiten Erfassungsschritt ein Wert einer mittels der Sensoreinheit erfassten Lichtänderung in einem definierten Spektralbereich mit einem Grenzwert verglichen und bei einer Unterschreitung des Grenzwerts der Blendschutzfilter aufgemacht wird. Vorzugsweise wird bei einer Unterschreitung des Grenzwerts die Abdunklung des Blendschutzfilters aufgehoben und der Blendschutzfilter aufgehellt. Besonders bevorzugt wird nach einer Abdunklung des Blendschutzfilters in dem zweiten Erfassungsschritt ein Wert einer mittels der Sensoreinheit erfassten Lichtintensitätsänderung, insbesondere verglichen mit dem Zustand vor der Abdunklung, in einem definierten Spektralbereich mit einem Grenzwert verglichen und bei einer Unterschreitung des Grenzwerts der Blendschutzfilter aufgemacht. Vorzugsweise definiert der Grenzwert dabei insbesondere eine definierte Änderung einer Lichtintensität in dem definierten Spektralbereich.

Unter einer "Blendschutzvorrichtung" soll in diesem Zusammenhang insbesondere eine Vorrichtung verstanden werden, welche zu einem Schutz eines Benutzers vor zu großer Helligkeit und/oder vor Funken vorgesehen ist. Vorzugsweise soll darunter insbesondere eine Vorrichtung verstanden werden, welche zu einem Schutz von Augen und/oder einem Gesichtsbereich eines Benutzers während eines Schweiß- und/oder Schleifprozesses dient. Bevorzugt soll darunter insbesondere eine Blendschutzvorrichtung verstanden werden, welche insbesondere zu einem Schutz der Augen eines Benutzers zumindest während eines Schweißprozesses dient. Es sind verschiedene, einem Fachmann als sinnvoll erscheinende Ausgestaltungen einer Blendschutzvorrichtung denkbar, wie beispielsweise als Schweißhelm, -schirm, -maske und/oder -schild. Unter einer "aktiven Blendschutzvorrichtung" soll in diesem Zusammenhang insbesondere eine Blendschutzvorrichtung mit einem aktiven optischen Blendschutzfilter verstanden werden.

Ferner soll dabei unter einem " aktiven optischen Blendschutzfilter" insbesondere ein optischer Filter verstanden werden, welcher insbesondere ein Schutzglas und/oder ein Kunststoffschutzglas bildet. Vorzugsweise soll darunter insbesondere ein optischer Filter verstanden werden, dessen Lichtdurchlässigkeit einstellbar ausgeführt ist. Bevorzugt soll darunter insbesondere ein optischer Schweißschutzfilter mit einer automatischen Verdunkelung verstanden werden. Besonders bevorzugt weist der Blendschutzfilter zumindest eine in der Transmission schaltbare Flüssigkristallebene auf. Es sind verschiedene, einem Fachmann als sinnvoll erscheinende Ausgestaltungen des optischen Blendschutzfilters denkbar, insbesondere soll darunter jedoch ein ADF, auch "automatic darkening filter" oder "automatischer Schweißerschutzfilter" genannt, verstanden werden. Unter einer "Sensoreinheit" soll in diesem Zusammenhang insbesondere eine Einheit verstanden werden, die dazu vorgesehen ist, zumindest eine Kenngröße und/oder eine physikalische Eigenschaft aufzunehmen, wobei die Aufnahme aktiv, wie insbesondere durch Erzeugen und Aussenden eines elektrischen Messsignals, und/oder passiv, wie insbesondere durch eine Erfassung von Eigenschaftsänderungen eines Sensorbauteils, stattfinden kann. Es sind verschiedene, einem Fachmann als sinnvoll erscheinende Sensoreinheiten denkbar. Vorzugsweise weist die Sensoreinheit insbesondere zumindest eine Fotozelle auf. Bevorzugt ist die Fotozelle insbesondere zumindest zu einer optischen Detektion eines Lichtbogens vorgesehen.

Ferner soll in diesem Zusammenhang unter einer "eingestellten Sensitivität" insbesondere eine auf einer Elektronik, insbesondere einer Steuer- und/oder Regeleinheit, der Blendschutzvorrichtung hinterlegte Einstellung bezüglich einer Abdunklungssensitivität verstanden werden. Vorzugsweise ist ein Wert und/oder einer Faktor der Abdunklungssensitivität mittels einer Bedieneinheit von einem Benutzer manuell einstellbar. Besonders bevorzugt umfasst die Sensitivitätseinstellung einen Grenzwert einer Intensität einer Lichtänderung, bei welchem eine Abdunklung des aktiven optischen Blendschutzfilters erfolgen soll. Unter einem "definierten Spektralbereich" soll in diesem Zusammenhang insbesondere ein bestimmter, begrenzter Spektralbereich eines einstrahlenden Lichts verstanden werden. Vorzugsweise soll darunter insbesondere ein definierter Wertebereich einer Wellenlänge eines einstrahlenden Lichts verstanden werden. Vorzugsweise umfasst der Wertebereich maximal 1000nm, vorzugsweise maximal 500nm eines Spektralbereichs. Unter "vorgesehen" soll insbesondere speziell programmiert, ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Durch das erfindungsgemäße Verfahren kann insbesondere eine vorteilhaft hohe Betriebssicherheit der Blendschutzvorrichtung erreicht werden. Es kann insbesondere zwischen einem Schweißbetrieb und einem Schleifbetrieb unterschieden werden und gleichzeitig eine hohe Betriebssicherheit erreicht werden. Insbesondere kann erreicht werden, dass ausreichend Zeit vorhanden ist, um zwischen einem Schweißbetrieb und einem Schleifbetrieb zu unterscheiden, ohne dadurch eine Betriebssicherheit zu beeinträchtigen, insbesondere da erst nach einem Abdunkeln entschieden wird, ob es sich um Schweiß- oder Schleiflicht handelt. Es wird daher insbesondere nicht beim Abdunkeln entschieden, ob es sich um Schweiß- oder Schleiflicht handelt, sondern erst unmittelbar nach dem Abdunkeln. Ferner kann dadurch die Blendschutzvorrichtung selbst erkennen, ob es sich um Schweißlicht oder Schleiflicht handelt. Es ist insbesondere keine manuelle Bedienung bzw. Umschaltung mehr notwendig. Hierdurch kann ein gefährliches Vergessen der Umschaltung vermieden werden.

Ferner wird vorgeschlagen, dass in dem zweiten Erfassungsschritt die Lichtänderung im sichtbaren Lichtspektrum mittels der Sensoreinheit erfasst wird. Vorzugsweise wird nach einer Abdunklung des Blendschutzfilters in dem zweiten Erfassungsschritt ein Wert einer mittels der Sensoreinheit erfassten Lichtänderung im sichtbaren Lichtspektrum mit einem Grenzwert verglichen und bei einer Unterschreitung des Grenzwerts der Blendschutzfilter aufgemacht. Es wird insbesondere eine Lichtänderung in einem Spektralbereich von 200 nm bis 1000 nm, vorzugsweise von 300 nm bis 800 nm und besonders bevorzugt von 380 nm bis 780 nm ermittelt. Dadurch kann vorteilhaft unterschieden werden, ob es sich bei dem einstrahlenden und/oder erfassten Licht um Schweiß- oder Schleiflicht handelt. Hierdurch kann eine besonders zuverlässige Unterscheidung erreicht werden.

Des Weiteren wird vorgeschlagen, dass in dem ersten Erfassungsschritt die Lichtänderung mittels der Sensoreinheit erfasst und verarbeitet wird. Vorzugsweise erfolgt eine Verarbeitung der Lichtänderung mittels einer Analogschaltung. Bevorzugt erfolgt eine Verarbeitung mittels einer elektronischen Schaltung, welche vorzugsweise Spannungs- und/oder Vergleichsregelbausteine umfasst. Bevorzugt erfolgt auch eine Signalübertragung analog. Hierdurch kann insbesondere eine vorteilhaft schnelle Informationsverarbeitung erreicht werden. Ferner kann dadurch eine vorteilhaft hohe Reaktionszeit der Blendschutzvorrichtung und damit eine schnelle Abdunklungszeit des Blendschutzfilters erreicht werden. Durch die Erfassung und analoge Verarbeitung kann insbesondere eine vorteilhaft schnelle und zuverlässige Abdunklung des Blendschutzfilters gewährleistet werden und damit eine hohe Betriebssicherheit erreicht werden.

Es wird ferner vorgeschlagen, dass in dem zweiten Erfassungsschritt die Lichtänderung in einem definierten Spektralbereich mittels der Sensoreinheit erfasst und digital verarbeitet wird. Vorzugsweise erfolgt eine Erfassung und/oder Verarbeitung der Lichtänderung mittels eines Mikroprozessors. Ferner wird vorgeschlagen, dass in dem zweiten Erfassungsschritt eine Unterscheidung der von der Sensoreinheit erfassten Lichtintensität in einem definierten Spektralbereich und dem Grenzwert mittels eines Mikroprozessors erfolgt. Bevorzugt benötigt die Unterscheidung der verschiedenen Licht-Emissionen die Rechenleistung eines Prozessors, insbesondere des Mikroprozessors. Abhängig von einem Mikroprozessor ist die dazu benötigte Zeit zwar im Rahmen der gemäß der Norm EN379 erlaubten 4 ms möglich, für einen realen Blendschutzfilter aber nicht brauchbar und/oder es werden leistungsstarke und damit teure Mikroprozessoren benötigt, um eine vertretbare Rechenzeit zu gewährleisten. Dadurch kann zuverlässig unterschieden werden, ob es sich bei dem einstrahlenden und/oder erfassten Licht um Schweiß- oder Schleiflicht handelt. Ferner kann dadurch ausreichend Zeit für eine Erfassung einer Lichtänderung in einem definierten Spektralbereich bereitgestellt werden. Hierdurch kann insbesondere auf einen teuren und schnellen Mikroprozessor verzichtet werden und/oder ein ungenaue Erfassung vermieden werden.

Erfindungsgemäß wird vorgeschlagen, dass in dem zweiten Erfassungsschritt nach einer Abdunklung des Blendschutzfilters zwischen einem Schleiflicht und einem Schweißlicht unterschieden wird, wobei bei einem erfassten Schleiflicht der Blendschutzfilter aufgemacht wird und bei einem erfassten Schweißlicht der Blendschutzfilter abgedunkelt belassen wird. Unter einem "Schleiflicht" soll in diesem Zusammenhang insbesondere ein durch Funken, insbesondere Schleifunken, welche bei einer Schleifbearbeitung erzeugt werden, entstehendes Licht verstanden werden. Vorzugsweise unterscheidet sich das Schleiflicht von einem Schweißlicht hinsichtlich eines Spektralbereichs des Lichts. Insbesondere unterscheidet sich das Schleiflicht von einem Schweißlicht in einer Lichtintensität in einem sichtbaren Lichtspektrum. Besonders bevorzugt weist das Schweißlicht in einem sichtbaren Lichtspektrum eine höhere Intensität auf. Dadurch kann insbesondere bei einem Schleifvorgang eine vorteilhafte Sicht gewährleistet werden. Es kann insbesondere auf eine manuelle Umschaltung zwischen einem Schleifbetrieb und einem Schweißbetrieb verzichtet werden. Hierdurch kann insbesondere auf ein zusätzliches Bedienelement zu einer manuellen Umschaltung zwischen einem Schleifbetrieb und einem Schweißbetrieb verzichtet werden.

Ferner geht die Erfindung aus von einer Blendschutzvorrichtung, die zu einer Durchführung des Verfahrens vorgesehen ist, mit einem aktiven optischen Blendschutzfilter, mit zumindest einer Sensoreinheit und mit zumindest einer mit der Sensoreinheit gekoppelten Steuer- und/oder Regeleinheit, welche dazu vorgesehen ist, abhängig von einem erfassten Arbeitszustand und/oder einer Lichteinstrahlung eine Durchlässigkeit des optischen Blendschutzfilters zu steuern und/oder zu regeln. Es wird vorgeschlagen, dass die Steuer- und/oder Regeleinheit zumindest einen Mikroprozessor aufweist, welcher zu einer Unterscheidung der mittels der Sensoreinheit erfassten Lichtintensität in einem definierten Spektralbereich und dem Grenzwert vorgesehen ist. Vorzugsweise ist der Mikroprozessor zu einer Unterscheidung der mittels der Sensoreinheit erfassten Lichtintensität in einem definierten Spektralbereich und dem Grenzwert dazu vorgesehen, Lichtänderungen gemäß ihrer Wellenlänge zu unterscheiden. Unter einer "Steuer- und/oder Regeleinheit" soll in diesem Zusammenhang insbesondere eine Einheit mit zumindest einer Steuerelektronik verstanden werden. Unter einer "Steuerelektronik" soll insbesondere eine Einheit mit zumindest einer elektronischen Schaltung, welche vorzugsweise aus Spannungs- und Vergleichsregelbausteinen besteht, und/oder mit einer Prozessoreinheit, insbesondere einem Mikroprozessor, und mit einer Speichereinheit sowie mit einem in der Speichereinheit gespeicherten Betriebsprogramm verstanden werden. Dadurch kann zuverlässig unterschieden werden, ob es sich bei dem einstrahlenden und/oder erfassten Licht um Schweiß- oder Schleiflicht handelt. Ferner kann dadurch ausreichend Zeit für eine Erfassung einer Lichtänderung in einem definierten Spektralbereich bereitgestellt werden. Hierdurch kann insbesondere eine vorteilhaft hohe Betriebssicherheit der Blendschutzvorrichtung erreicht werden.

Des Weiteren wird vorgeschlagen, dass die Sensoreinheit zumindest einen Tageslichtsensor aufweist, der zu einer Erfassung von Lichtänderungen im sichtbaren Lichtspektrum vorgesehen ist. Vorzugsweise ist der Tageslichtsensor insbesondere dazu vorgesehen, eine Intensität eines einstrahlenden und/oder erfassten Lichts im sichtbaren Lichtspektrum, also insbesondere zwischen 380 nm und 780 nm, zu erfassen. Bevorzugt ist der Tageslichtsensor insbesondere dazu vorgesehen, eine Intensität eines einstrahlenden und/oder erfassten Lichts ausschließlich im sichtbaren Lichtspektrum, also insbesondere zwischen 380 nm und 780 nm, zu erfassen. Unter einem "Tageslichtsensor" soll in diesem Zusammenhang insbesondere ein Sensor verstanden werden, der, insbesondere ausschließlich, auf Licht im sichtbaren Spektrum reagiert. Vorzugsweise ist der Sensor insbesondere zu einer ausschließlichen Lichterfassung in einem definierten Spektrum vorgesehen. Durch den Tageslichtsensor kann vorteilhaft zuverlässig unterschieden werden, ob es sich bei dem einstrahlenden und/oder erfassten Licht um Schweiß- oder Schleiflicht handelt. Hierdurch kann eine besonders zuverlässige Unterscheidung erreicht werden.

Es wird ferner vorgeschlagen, dass die Sensoreinheit zumindest einen von einer Photozelle gebildeten Sensor aufweist, welcher zu einer Erfassung einer Intensität einer Lichteinstrahlung vorgesehen ist. Dadurch kann ein vorteilhaft zuverlässiger Sensor mit einer vorteilhaft hohen Energieausbeute bereitgestellt werden. Ferner kann insbesondere auf eine externe Energieversorgung wie beispielsweise mittels einer Batterie und/oder mittels eines Akkumulators verzichtet werden. Bei der Verwendung einer Photozelle kann insbesondere eine Sensoreinheit bereitgestellt werden, mittels welcher insbesondere auch Licht in einem nicht sichtbaren Bereich, wie insbesondere Infrarotlicht, erfasst und vorzugsweise verwertet werden kann. Es kann insbesondere zuverlässig eine Erfassung einer Intensität einer Lichteinstrahlung erreicht werden. Hierdurch kann wiederum eine zuverlässig schnelle und sichere Abdunklung erreicht werden.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine Blendschutzvorrichtung mit einem aktiven optischen Blendschutzfilter, mit zumindest einer Sensoreinheit und mit zumindest einer mit der Sensoreinheit gekoppelten Steuer- und/oder Regeleinheit in einer schematischen Darstellung und
- Fig. 2: ein schematisches Ablaufdiagramm eines erfindungsgemäßen Verfahrens zu einem Betrieb der Blendschutzvorrichtung.

### Beschreibung des Ausführungsbeispiels

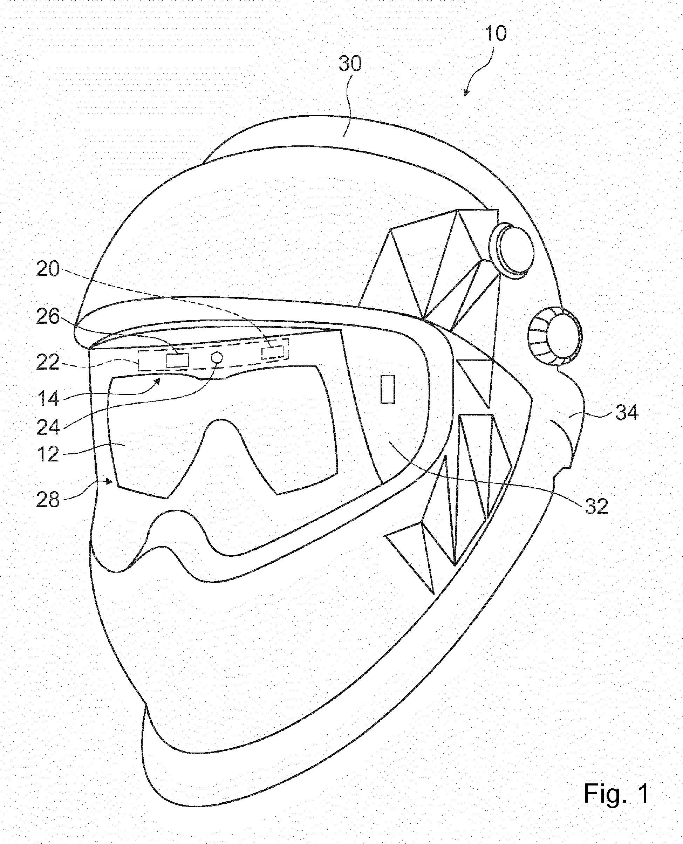

Die Figur 1 zeigt eine Blendschutzvorrichtung 10. Die Blendschutzvorrichtung 10 ist dazu vorgesehen, während eines Betriebs von einem Benutzer auf dem Kopf getragen zu werden. Der Benutzer ist hierbei von einem Träger gebildet. Figur 1 zeigt die Blendschutzvorrichtung 10 in einer Betriebsstellung. Die Blendschutzvorrichtung 10 ist von einem Schweißhelm gebildet. Grundsätzlich wäre jedoch auch eine andere, einem Fachmann als sinnvoll erscheinende Ausbildung der Blendschutzvorrichtung 10 denkbar.

Die Blendschutzvorrichtung 10 weist einen aktiven optischen Blendschutzfilter 12 auf. Ferner weist die Blendschutzvorrichtung 10 eine Sensoreinheit 14 auf. Die Sensoreinheit 14 ist zu einer Erfassung eines Arbeitszustands und einer davon abhängigen Ansteuerung einer Durchlässigkeit des Blendschutzfilters 12 vorgesehen. Die Sensoreinheit 14 weist zumindest einen Sensor 26 auf, der dazu vorgesehen ist, einen Schweißvorgang oder das Auftreten eines hellen Lichts, welches die Augen eines Benutzers schädigen oder auf andere Weise beeinflussen könnte, zu detektieren. Die Sensoreinheit 14 weist einen von einer Photozelle gebildeten Sensor 26 auf, welcher zu einer Erfassung einer Intensität einer Lichteinstrahlung vorgesehen ist. Grundsätzlich wäre jedoch auch eine andere, einem Fachmann als sinnvoll erscheinende Ausbildung des Sensors 26 der Sensoreinheit 14 denkbar. Der Sensor 26 ist zu einer allgemeinen Erfassung einer Intensität einer Lichteinstrahlung vorgesehen. Der Sensor 26 ist von einem IR-Sensor gebildet. Ferner weist die Sensoreinheit 14 einen Tageslichtsensor 24 auf, der zu einer Erfassung von Lichtänderungen im sichtbaren Lichtspektrum vorgesehen ist. Der Tageslichtsensor 24 ist dazu vorgesehen, eine Intensität eines einstrahlenden und eines erfassten Lichts im sichtbaren Lichtspektrum, also zwischen 380 nm und 780 nm, zu erfassen. Der Tageslichtsensor 24 ist dazu vorgesehen, eine Intensität eines einstrahlenden und erfassten Lichts ausschließlich im sichtbaren Lichtspektrum zu erfassen. Der Tageslichtsensor 24 ist ebenfalls von einer Photozelle gebildet. Grundsätzlich wäre jedoch auch eine andere, einem Fachmann als sinnvoll erscheinende Ausbildung des Tageslichtsensors 24 denkbar.

Des Weiteren weist die Blendschutzvorrichtung 10 eine Schutzkassette 28 auf. Der aktive optische Blendschutzfilter 12 und die Sensoreinheit 14 bilden einen Teil der Schutzkassette 28. Ferner weist die Blendschutzvorrichtung 10 eine mit der Sensoreinheit 14 gekoppelte Steuer- und Regeleinheit 22 auf. Die Steuer- und Regeleinheit 22 ist dazu vorgesehen, abhängig von einem erfassten Arbeitszustand und einer Lichteinstrahlung eine Durchlässigkeit des optischen Blendschutzfilters 12 zu steuern. Die Steuer- und Regeleinheit 22 ist dazu vorgesehen, die Daten der Sensoreinheit 14 zu verarbeiten und abhängig davon den Blendschutzfilter 12 anzusteuern. Die Steuer- und Regeleinheit 22 umfasst zu einer Energieversorgung eine nicht weiter sichtbare Batterie und/oder Solarzelle. Ferner weist die Steuer- und Regeleinheit 22 einen Mikroprozessor 20 auf. Der Mikroprozessor 20 ist zu einer Unterscheidung der mittels der Sensoreinheit 14 erfassten Lichtintensität in einem definierten Spektralbereich und dem Grenzwert vorgesehen. Der Mikroprozessor 20 ist zu einer Auswertung der Daten des Tageslichtsensors 24 vorgesehen. Der Mikroprozessor 20 ist dazu vorgesehen, abhängig von den Daten des Tageslichtsensors 24 eine Intensitätsänderung eines erfassten Lichts abhängig von einer Wellenlänge zu erfassen und auszuwerten. Ferner ist der Mikroprozessor 20 dazu vorgesehen, nach einer Abdunklung des Blendschutzfilters 12 zwischen einem Schleiflicht und einem Schweißlicht zu unterscheiden. Die Steuer- und Regeleinheit 22 ist dazu vorgesehen, den Blendschutzfilter 12 abzudunkeln, wenn über den Sensor 26 der Sensoreinheit 14 eine Lichtänderung größer gleich einer eingestellten Sensitivität erfasst wird, und anschließend mittels den Daten des Tageslichtsensors 24 mittels des Mikroprozessors 20 zwischen einem Schleiflicht und einem Schweißlicht zu unterscheiden. Bei einem erfassten Schleiflicht ist die Steuer- und Regeleinheit 22 dazu vorgesehen, den Blendschutzfilter 12 aufzumachen und bei einem erfassten Schweißlicht den Blendschutzfilter 12 abgedunkelt zu belassen.

Der aktive optische Blendschutzfilter 12 ist von einem elektrooptischen Filter gebildet. Der aktive optische Blendschutzfilter 12 ist von einem automatic darkening filter, kurz ADF, gebildet. Der optische Blendschutzfilter 12 besteht aus mehreren Schichten. Der optische Blendschutzfilter 12 ist als ein Mehrschichtverbund ausgebildet. Der optische Blendschutzfilter 12 weist eine Flüssigkristallschicht auf, welche als eine Blende des Blendschutzfilters 12 dient. Die Flüssigkristallschicht ist von einem transluzenten Flüssigkristallbildschirm gebildet. Die Flüssigkristallschicht wird von der Steuer- und Regeleinheit 22 angesteuert. Die Flüssigkristallschicht des Blendschutzfilters 12 wird von der Steuer- und Regeleinheit 22 aktiviert, wenn über die Sensoreinheit 14 eine Lichtänderung größer gleich einer eingestellten Sensitivität erfasst wird.

Der Blendschutzfilter 12 weist eine rechteckige Grundform auf. Ferner weist der Blendschutzfilter 12 einen Nasenausschnitt auf. Der Nasenausschnitt ist von einer immateriellen Aussparung in einem materiellen Teil des Blendschutzfilters 12 gebildet. Der Nasenausschnitt ist von einer immateriellen Aussparung in einem materiellen, zumindest teilweise transluzenten Teilbereich des Blendschutzfilters 12 gebildet.

Ferner weist die Blendschutzvorrichtung 10 eine Schildeinheit 30 auf. Der Blendschutzfilter 12 ist fest in der Schildeinheit 30 aufgenommen. Der Blendschutzfilter 12 ist positionsfest in der Schildeinheit 30 aufgenommen. Der Blendschutzfilter 12 ist in einer Ausnehmung in der Schildeinheit 30 eingepasst. Die gesamte Schutzkassette 28 ist in einer Ausnehmung in der Schildeinheit 30 eingepasst. Die Schildeinheit 30 besteht aus einem im Wesentlichen formfesten Material. Die Schildeinheit 30 besteht aus einem Kunststoff, welcher insbesondere beständig gegenüber Funken und/oder anderen, beim Schweißen auftretenden Einflüssen ist. Die Blendschutzvorrichtung 10 weist ferner eine Vorsatzscheibe 32 auf. Die Vorsatzscheibe 32 ist mit der Schildeinheit 30 über nicht weiter sichtbare Rastelemente verbunden. Des Weiteren weist die Blendschutzvorrichtung 10 eine Kopfbefestigungseinheit 34 auf. Die Kopfbefestigungseinheit 34 ist zu einer Befestigung an dem Kopf des Benutzers vorgesehen. Die Kopfbefestigungseinheit 34 ist von einem Kopfband gebildet.

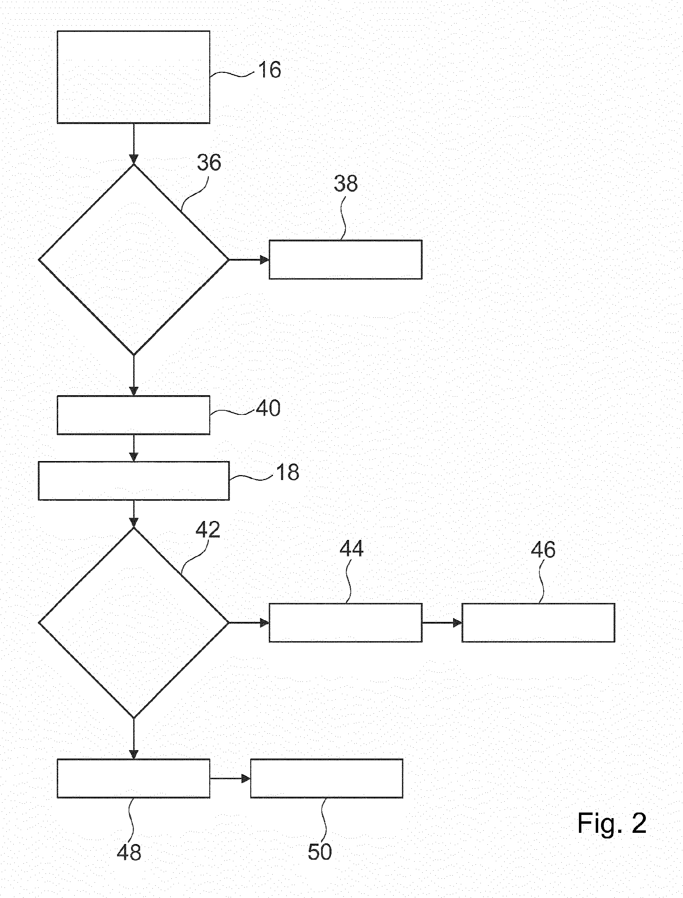

Figur 2 zeigt ein schematisches Ablaufdiagramm eines Verfahrens zu einem Betrieb der Blendschutzvorrichtung 10. Die Blendschutzvorrichtung 10 ist zu einer Durchführung des Verfahrens vorgesehen. Das Verfahren dient einer automatischen Unterscheidung zwischen einem Schleiflicht und einem Schweißlicht und einer entsprechenden Einstellung des Blendschutzfilters 12.

In dem Verfahren wird mittels der Sensoreinheit 14 in einem ersten Erfassungsschritt 16 eine Lichtänderung erkannt. In dem ersten Erfassungsschritt 16 wird die Lichtänderung mittels der Sensoreinheit 14 erfasst und verarbeitet. Eine Verarbeitung erfolgt insbesondere analog. In dem ersten Erfassungsschritt 16 wird die Lichtänderung mittels des Sensors 26 der Sensoreinheit 14 erkannt. Anschließend werden die Signale des Sensors 26 von der Steuer und Regeleinheit 22 verarbeitet. Die Signale des Sensors 26 werden von der Steuer und Regeleinheit 22 analog verarbeitet. Abhängig von einer Intensität der Lichtänderung erfolgt eine davon abhängige Ansteuerung einer Durchlässigkeit des Blendschutzfilters 12, wobei der Blendschutzfilter 12 bei einer Lichtänderung größer gleich einer eingestellten Sensitivität abgedunkelt wird. In einer Verzweigung 36 wird dazu mittels der Steuer- und Regeleinheit 22 überprüft, ob die mittels des Sensors 26 erfasste Lichtänderung größer als eine Sensitivitätseinstellung ist. Ist die mittels des Sensors 26 erfasste Lichtänderung nicht größer als eine Sensitivitätseinstellung, erfolgt in einem weiteren Verfahrensschritt 38 keine weitere Aktion und der erste Erfassungsschritt 16 wird wiederholt. Ist die mittels des Sensors 26 erfasste Lichtänderung größer als eine Sensitivitätseinstellung, wird der Blendschutzfilter 12 in einem weiteren Verfahrensschritt 40 abgedunkelt. Nach einer Abdunklung des Blendschutzfilters 12 wird in einem zweiten Erfassungsschritt 18 ein Wert einer mittels der Sensoreinheit 14 erfassten Lichtänderung in einem definierten Spektralbereich mit einem Grenzwert verglichen. In dem zweiten Erfassungsschritt 18 wird die Lichtänderung im sichtbaren Lichtspektrum mittels der Sensoreinheit 14 erfasst. Die Lichtintensitätsänderung im sichtbaren Lichtspektrum wird mittels des Tageslichtsensors 24 der Sensoreinheit 14 erfasst. In dem zweiten Erfassungsschritt 18 wird die Lichtänderung in dem definierten Spektralbereich mittels der Sensoreinheit 14 erfasst und digital verarbeitet. In dem zweiten Erfassungsschritt 18 erfolgt eine Unterscheidung der von der Sensoreinheit 14 erfassten Lichtintensität in einem definierten Spektralbereich und dem Grenzwert mittels eines Mikroprozessors 20. In dem zweiten Erfassungsschritt 18 erfolgt insbesondere eine Messung einer Intensität des Tageslichts. In dem zweiten Erfassungsschritt 18 wird nach einer Abdunklung des Blendschutzfilters 12 zwischen einem Schleiflicht und einem Schweißlicht unterschieden, wobei bei einem erfassten Schleiflicht der Blendschutzfilter 12 aufgemacht wird und bei einem erfassten Schweißlicht der Blendschutzfilter 12 abgedunkelt belassen wird. In einer Verzweigung 42 wird dazu mittels der Steuer- und Regeleinheit 22 überprüft, ob eine Wertänderung des Tageslichtsensors 24 der Sensoreinheit 14 größer als ein hinterlegter Grenzwert ist. Der Grenzwert definiert dabei eine definierte Änderung einer Lichtintensität in dem sichtbaren Spektralbereich. Bei einer Unterschreitung des Grenzwerts wird der Blendschutzfilter 12 in einem weiteren Verfahrensschritt 44 aufgemacht. Anschließend wird der Blendschutzfilter 12 in einem Verfahrensschritt 46 in den Modus "Schleifen" geschalten. Ist die Wertänderung des Tageslichtsensors 24 der Sensoreinheit 14 größer als ein hinterlegter Grenzwert, wird der Blendschutzfilter 12 in einem weiteren Verfahrensschritt 48 abgedunkelt belassen. Anschließend wird der Blendschutzfilter 12 in einem Verfahrensschritt 50 in den Modus "Schweißen" geschalten.

### Bezugszeichen

- 10: Blendschutzvorrichtung
- 12: Blendschutzfilter
- 14: Sensoreinheit
- 16: Erfassungsschritt
- 18: Erfassungsschritt
- 20: Mikroprozessor
- 22: Steuer- und/oder Regeleinheit
- 24: Tageslichtsensor
- 26: Sensor
- 28: Schutzkassette
- 30: Schildeinheit
- 32: Vorsatzscheibe
- 34: Kopfbefestigungseinheit
- 36: Verzweigung
- 38: Verfahrensschritt
- 40: Verfahrensschritt
- 42: Verzweigung
- 44: Verfahrensschritt
- 46: Verfahrensschritt
- 48: Verfahrensschritt
- 50: Verfahrensschritt

## Patentansprüche

1. Verfahren zu einem Betrieb einer Blendschutzvorrichtung (10) mit einem aktiven optischen Blendschutzfilter (12) und mit zumindest einer Sensoreinheit (14), wobei mittels der Sensoreinheit (14) in einem ersten Erfassungsschritt (16) eine Lichtänderung erkannt wird und abhängig von einer Intensität der Lichtänderung eine davon abhängige Ansteuerung einer Durchlässigkeit des Blendschutzfilters (12) erfolgt, wobei der Blendschutzfilter (12) bei einer Lichtänderung größer gleich einer eingestellten Sensitivität abgedunkelt wird,
wobei
nach einer Abdunklung des Blendschutzfilters (12) in einem zweiten Erfassungsschritt (18) ein Wert einer mittels der Sensoreinheit (14) erfassten Lichtänderung in einem definierten Spektralbereich mit einem Grenzwert verglichen und bei einer Unterschreitung des Grenzwerts der Blendschutzfilter (12) aufgemacht wird, **dadurch gekennzeichnet, dass** in dem zweiten Erfassungsschritt (18) nach einer Abdunklung des Blendschutzfilters (12) zwischen einem Schleiflicht und einem Schweißlicht unterschieden wird, wobei bei einem erfassten Schleiflicht der Blendschutzfilter (12) aufgemacht wird und bei einem erfassten Schweißlicht der Blendschutzfilter (12) abgedunkelt belassen wird.

2. Verfahren nach Anspruch 1,
**dadurch gekennzeichnet, dass**
in dem zweiten Erfassungsschritt (18) die Lichtänderung im sichtbaren Lichtspektrum mittels der Sensoreinheit (14) erfasst wird.

3. Verfahren nach Anspruch 1 oder 2,
**dadurch gekennzeichnet, dass**
in dem ersten Erfassungsschritt (16) die Lichtänderung mittels der Sensoreinheit (14) erfasst und verarbeitet wird.

4. Verfahren nach einem der vorhergehenden Ansprüche,
**dadurch gekennzeichnet, dass**
in dem zweiten Erfassungsschritt (18) die Lichtänderung in einem definierten Spektralbereich mittels der Sensoreinheit (14) erfasst und digital verarbeitet wird.

5. Verfahren nach Anspruch 4,
**dadurch gekennzeichnet, dass**
in dem zweiten Erfassungsschritt (18) eine Unterscheidung der von der Sensoreinheit (14) erfassten Lichtintensität in einem definierten Spektralbereich und dem Grenzwert mittels eines Mikroprozessors (20) erfolgt.

6. Blendschutzvorrichtung, die zu einer Durchführung des Verfahrens nach einem der vorhergehenden Ansprüche vorgesehen ist, mit einem aktiven optischen Blendschutzfilter (12), mit zumindest einer Sensoreinheit (14) und mit zumindest einer mit der Sensoreinheit (14) gekoppelten Steuer- und/oder Regeleinheit (22), welche dazu vorgesehen ist, abhängig von einem erfassten Arbeitszustand und/oder einer Lichteinstrahlung eine Durchlässigkeit des optischen Blendschutzfilters (12) zu steuern und/oder zu regeln.

7. Blendschutzvorrichtung nach Anspruch 6,
**dadurch gekennzeichnet, dass**
die Steuer- und/oder Regeleinheit (22) zumindest einen Mikroprozessor (20) aufweist, welcher zu einer Unterscheidung der mittels der Sensoreinheit (14) erfassten Lichtintensität in einem definierten Spektralbereich und dem Grenzwert vorgesehen ist.

8. Blendschutzvorrichtung nach Anspruch 6 oder 7,
**dadurch gekennzeichnet, dass**
die Sensoreinheit (14) zumindest einen Tageslichtsensor (24) aufweist, der zu einer Erfassung von Lichtänderungen im sichtbaren Lichtspektrum vorgesehen ist.

9. Blendschutzvorrichtung nach einem der Ansprüche 6 bis 8,
**dadurch gekennzeichnet, dass**
die Sensoreinheit (14) zumindest einen von einer Photozelle gebildeten Sensor (26) aufweist, welcher zu einer Erfassung einer Intensität einer Lichteinstrahlung vorgesehen ist.

## Claims

1. Method for operating a glare protection device (10)
with an active optical glare protection filter (12) and
with at least one sensor unit (14),
wherein a change of light is detected by means of the sensor unit (14) in a first detection step (16) and - as a function of an intensity of the change of light - a transmittance of the glare protection filter (12) is actuated depending on the detection of the change of light,
wherein the glare protection filter (12) is darkened if there is a change of light greater than or equal to a set sensitivity,
wherein after a darkening of the glare protection filter (12), in a second detection step (18), a value of a change of light in a defined spectral range, detected by means of the sensor unit (14), is compared with a limit value and the glare protection filter (12) is opened if the limit value is undershot,
**characterized in that**
in the second detection step (18), after the darkening of the glare protection filter (12), a distinction is made between a grinding light and a welding light,
wherein if a grinding light is detected, the glare protection filter (12) is opened and if a welding light is detected, the glare protection filter (12) is left in the darkened state.

2. Method according to claim 1,
**characterized in that**
in the second detection step (18), the change of light in the visible light spectrum is detected by means of the sensor unit (14).

3. Method according to claim 1 or 2,
**characterized in that**
in the first detection step (16), the change of light is detected and processed by means of the sensor unit (14).

4. Method according to one of the preceding claims,
**characterized in that**
in the second detection step (18), the change of light in a defined spectral range is detected and digitally processed by means of the sensor unit (14).

5. Method according to claim 4,
**characterized in that**
in the second detection step (18), a distinction is made between the light intensity in a defined spectral range, detected by the sensor unit (14), and the limit value by means of a microprocessor (20).

6. Glare protection device which is configured for carrying out the method according to one of the preceding claims,
with an active optical glare protection filter (12),
with at least one sensor unit (14) and
with at least one control and/or regulation unit (22), which is coupled with the sensor unit (14) and is configured to control and/or regulate a transmittance of the optical glare protection filter (12) depending on a detected operative state and/or a light irradiation.

7. Glare protection device according to claim 6,
**characterized in that**
the control unit and/or regulation unit (22) comprises at least one microprocessor (20), which is configured for a distinction between the light intensity in a defined spectral range, detected by means of the sensor unit (14), and the limit value.

8. Glare protection device according to claim 6 or 7,
**characterized in that**
the sensor unit (14) comprises at least one daylight sensor (24), which is configured to detect changes of light in the visible light spectrum.

9. Glare protection device according to one of claims 6 to 8,
**characterized in that**
the sensor unit (14) comprises at least one sensor (26), which is realized as a photocell and is configured for a detection of an intensity of a light irradiation.

## Revendications

1. Procédé pour faire fonctionner un dispositif anti-éblouissement (10) avec un filtre anti-éblouissement optique actif (12) et
avec au moins une unité de capteur (14),
où un changement de la lumière est détecté moyennant l'unité de capteur (14) dans une première étape de détection (16) et où - en fonction d'une intensité du changement de lumière - un actionnement d'une transmittance du filtre anti-éblouissement (12) en fonction de ladite détection du changement de lumière est effectuée,
où le filtre anti-éblouissement (12) s'assombri lors d'un changement de lumière supérieur ou égal à une sensibilité réglée,
où après un assombrissement du filtre anti-éblouissement (12), dans une deuxième étape de détection (18), une valeur d'un changement de lumière dans une plage spectrale définie, détecté moyennant l'unité de capteur (14), est comparée à une valeur limite et, en cas de sous-dépassement de la valeur limite, le filtre anti-éblouissement (12) est ouvert,
**caractérisé en ce que**
dans la deuxième étape de détection (18), après un assombrissement du filtre anti-éblouissement (12), une distinction est faite entre une lumière de ponçage et une lumière de soudage, où en cas de lumière de ponçage détectée, le filtre anti-éblouissement (12) est ouvert et en cas de lumière de soudage détectée, le filtre anti-éblouissement (12) est laissé assombri.

2. Procédé selon la revendication 1,
**caractérisé en ce que**
dans la deuxième étape de détection (18), le changement de lumière dans le spectre de lumière visible est détecté moyennant l'unité de capteur (14).

3. Procédé selon la revendication 1 ou 2,
**caractérisé en ce que**
dans la première étape de détection (16), le changement de lumière est détecté et traité moyennant l'unité de capteur (14).

4. Procédé selon l'une des revendications précédentes,
**caractérisé en ce que**
dans la deuxième étape de détection (18), le changement de lumière dans une plage spectrale définie est détecté et traité numériquement moyennant l'unité de capteur (14).

5. Procédé selon la revendication 4,
**caractérisé en ce que**
dans la deuxième étape de détection (18), une distinction entre l'intensité de lumière dans une plage spectrale définie, détectée moyennant l'unité de capteur (14), et la valeur limite est effectuée moyennant un microprocesseur (20).

6. Dispositif anti-éblouissement prévu pour mettre en oeuvre le procédé selon l'une des revendications précédentes,
avec un filtre anti-éblouissement optique actif (12),
avec au moins une unité de capteur (14) et
avec au moins une unité de commande et/ou de régulation (22) qui est couplée à l'unité de capteur (14) et est prévue pour commander et/ou réguler une transmittance du filtre anti-éblouissement optique (12) en fonction d'un état opératif détecté et/ou d'une irradiation lumineuse.

7. Dispositif anti-éblouissement selon la revendication 6,
**caractérisé en ce que**
l'unité de commande et/ou de régulation (22) comprend au moins un microprocesseur (20) qui est prévu pour distinguer entre l'intensité de lumière dans une plage spectrale définie, détectée moyennant l'unité de capteur (14), et la valeur limite.

8. Dispositif anti-éblouissement selon la revendication 6 ou 7,
**caractérisé en ce que**
l'unité de capteur (14) comprend au moins un capteur de lumière du jour (24) prévu pour détecter des changements de lumière dans le spectre de lumière visible.

9. Dispositif anti-éblouissement selon l'une des revendications 6 à 8,
**caractérisé en ce que**
l'unité de capteur (14) comprend au moins un capteur (26) qui est réalisé d'une cellule photoélectrique et est prévu pour détecter une intensité d'une irradiation lumineuse.
